# NEUE EUROPÄISCHE PATENTSCHRIFT

(11) **EP 0 688 549 B2**
(45) Veröffentlichungstag und Bekanntmachung des Hinweises auf die Entscheidung über den Einspruch: **01.03.2006**
(45) Hinweis auf die Patenterteilung: 10.10.2001
(21) Anmeldenummer: 95109533.0
(22) Anmeldetag: 20.06.1995
(51) Int. Cl.: A61F 13/15

(54) **Damenbinde**
Sanitary napkin
Serviette hygiènique

(30) Priorität: 20.06.1994 JP 13737694
(43) Veröffentlichungstag der Anmeldung: 27.12.1995
(73) Patentinhaber: UNI-CHARM CORPORATION, Kawanoe-shi Ehime-ken (JP)
(72) Erfinder: Wada, Ichiro, Kawanoe-shi, Ehime-ken (JP); Hisada, Kenichi, Kawanoe-shi, Ehime-ken (JP)
(74) Vertreter: Sperling, Rüdiger

(56) Entgegenhaltungen:
- EP-A- 0 091 412
- EP-A- 0 238 053
- WO-A-93/19711
- DE-A- 3 718 076
- DE-A- 4 004 729
- US-A- 4 556 146
- US-A- 4 772 282
- US-A- 4 802 884
- US-A- 5 295 988

## Beschreibung

Die vorliegende Erfindung betrifft eine Damenbinde und insbesondere eine Damenbinde oder Menstruationseinlage und dergleichen, die einzeln in einer Umhüllung verpackt ist.

Herkömmliche Damenbinden sind allgemein einzeln in einer Umhüllung zum Vertrieb an den Verbraucher verpackt. Wie beispielsweise in der japanischen offengelegten Gebrauchsmusteranmeldung Nr. 1993-92430 aufgezeigt, haben einige Binden an ihren einander seitlich gegenüberliegenden Seiten Sperrklappen mit elastischen Elementen, die unter einer Spannung stehen, die in Längsrichtung der Binde ausgeübt wird, so daß die Klappen normalerweise so vorgespannt werden können, daß sie sich zusammenziehen. Gemäß der in diesem Dokument zum Stand der Technik aufgezeigten Technik ziehen sich die Klappen zusammen und erheben sich auf der hautberührenden Oberfläche der Binde, um so die Sperren zu bilden, die verhindern, daß Menstruationsflüssigkeit beim Tragen der Binde seitlich austritt, und die in Längsrichtung der Binde nach innen gekrümmt sind.

Auch ist es möglich, die bekannte Binde, die die genannten Sperrklappen aufweist, wie vorstehend beschrieben, zwei- oder dreifach zu falten und anschließend einzeln zum Vertrieb an den Verbraucher zu verpacken. Es versteht sich von selbst, daß die Binde mit einem empfindlichen Körperteil einerTrägerin in Berührung gebracht wird und daher muß die Elastizität der Klappen gemäßigt sein, um die Gefahr zu vermeiden, daß die Klappen in die Haut des empfindlichen Körperteils einschneiden könnten. Die Binde ist jedoch, bedingt durch das Vorhandensein des flussigkeitssaugfähigen Kerns mit einer komprimierten Faserpulpeschicht, relativ starr, so daß dann, wenn die Elastizität der Klappen übermäßig gering ist, die Starrheit der Binde die Elastizität übertreffen könnte und so die Klappen daran hindern könnte, in ausreichender Weise sich zusammenzuziehen und auf der hautberührenden Oberfläche der Binde sich zu erheben. Folglich tritt die Gefahr auf, daß die Klappen nicht in der Lage sein konnten, zuverlassig das seitliche Austreten von Menstruationsflussigkeit zu verhindern.

Aus der deutschen Offenlegungsschrift DE 27 18 076 ist eine Menstruations- und Inkontinenzbinde mit einer flussigkeitsdurchlässigen Decklage, einer flüssigkeitsundurchlässigen Außenlage, einem zwischen diesen beiden Lagen angeordneten fiüssigkeitssaugfähigen Kern und einem Paar in Längsrichtung dehnbarer Sperrklappen bekannt, die auf beiden Seiten der Binde angeordnet sind, wobei sich die Sperrklappen aufgrund ihrer Elastizität aufrichten und ein Austreten von Körperflüssigkeiten verhindem. Auch hierbei besteht jedoch das Problem, daß das Aufrichten der Sperrklappen allein durch deren Elastizität erreicht wird, so daß bei der bekannten Menstruations- und Inkontinenzbinde ein Zielkonflikt besteht. So ist einerseits eine möglichst große Elastizität der Sperrklappen wünschenswert, um diese aufzurichten und einen möglichst guten Abdichtungseffekt zu erreichen. Andererseits kann eine große Elastizität der Sperrklappen zu einem Einschneiden in die Körperoberfläche führen.

Der Erfindung liegt somit die Aufgabe zugrunde, die eingangs beschriebenen bekannten Binden dahingehend zu verbessern, daß sich die Sperrklappen wirksam aufrichten, ohne in die Körperoberfläche einzuschneiden.

Diese Aufgabe wird, ausgehend von der eingangs beschriebenen bekannten Binde gemäß dem Oberbegriff des Anspruchs 1, durch die kennzeichnenden Merkmale des Anspruchs 1 gelöst.

Die erfindungsgemäße Binde umfaßt eine flüssigkeitsdurchlässige Decklage, eine flüssigkeitsundurchlässige Außenlage, einen zwischen diesen beiden Lagen angeordneten flüssigkeitssaugfähigen Kern und ein Paar in Längsrichtung dehnbare Sperrklappen, die auf beiden Seiten der Binde vorgesehen sind, wobei die Binde durch Zurückfalten von in Längsrichtung einander gegenuberliegenden Endabschnitten derselben entlang quer zu der Binde verlaufenden Faltlinien in drei Schichten gefaltet ist und anschließend in einer Umhüllung verpackt ist, wobei:
jede der Sperrklappen einen in Längsrichtung dehnbaren Abschnitt und nicht dehnbare Abschnitte umfaßt, die sich kontinuierlich von in Längsrichtung einander gegenüberliegenden Enden des dehnbaren Abschnitts erstrecken; und
der dehnbare Abschnitt eine Länge von 37 bis 70% bezüglich der Gesamtlänge der Damenbinde hat und die Faltlinien sich quer über den dehnbaren Abschnitt erstrecken.

Bei der auf diese Weise angeordneten Damenbinde neigt der dehnbare Abschnitt der Klappe dazu, sich zusammenzuziehen, wenn die Binde aus der Umhüllung entnommen wird und in Längsrichtung entfaltet wird. Genauer gesagt hat die in drei Schichten gefaltete Binde eine Restspannung der Faltung entlang den Faltlinien, unter deren Wirkung die in Längsrichtung einander gegenüberliegenden Endabschnitte der Binde dann, wenn der in Längsrichtung mittlere Abschnitt der gefalteten Binde auf einer horizontalen Oberfläche angeordnet wird, in einem leicht erhöhten Zustand entfaltet sind. Gleichzeitig zieht sich der dehnbare Abschnitt jeder Klappe zwischen ihren in Längsrichtung gegenüberliegenden Enden zusammen und erhebt sich auf der hautberührenden Oberfläche der Binde,
Fig. 1 ist eine perspektivische Ansicht einer einzeln verpackten Damenbinde;
Fig. 2 ist eine Draufsicht, die eine entfaltete Damenbinde teilweise ausgebrochen zeigt;
Fig. 3 ist eine Schnittansicht entlang einer Linie X-X in Fig. 2;
Fig. 4 ist eine perspektivische Ansicht einer auf ei-ner horizontalen Oberfläche angeordneten Damenbinde; und
Fig. 5 ist eine Schnittansicht entlang einer Linie Y-Y in Fig. 4.

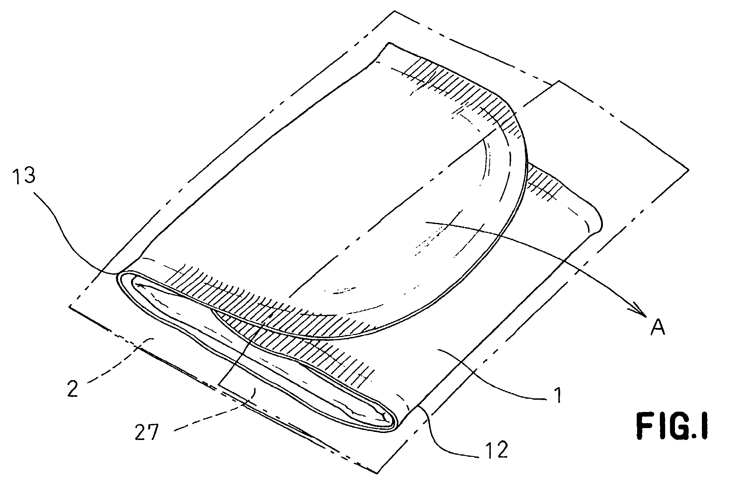

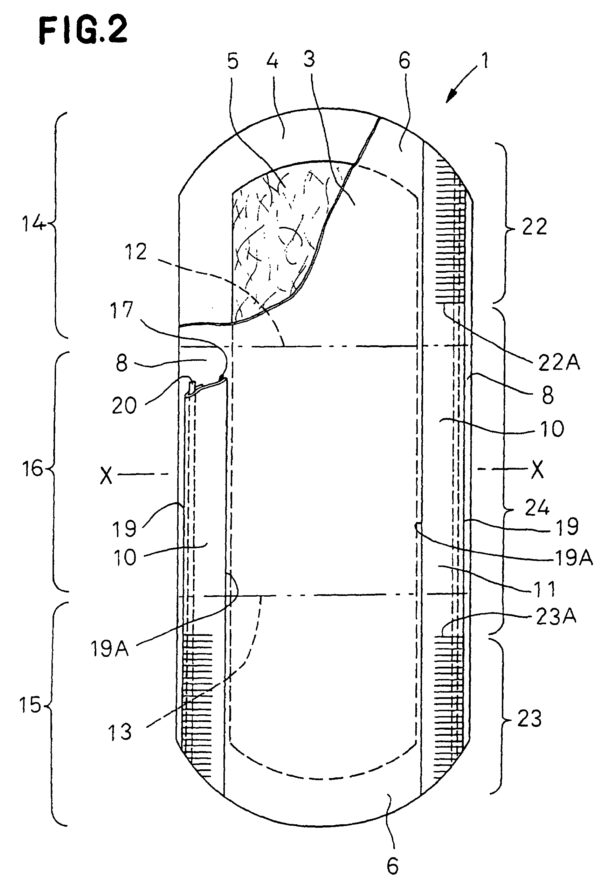

Wie Fig. 1 zeigt, ist eine Damenbinde 1 in Längsrichtung länglich geformt und in drei Schichten gefaltet und einzeln in einer Umhüllung 2 verpackt. In Fig. 2 ist die Damenbinde 1 aus der Umhüllung 2 entnommen und horizontal entfaltet. Die Binde 1 umfaßt eineflüssigkeitsdurchlässige Decklage 3, die aus einem Vliesstoff aus thermoplastischen Synthetikfasern hergestellt ist, eine flüssigkeitsundurchlässige Außenlage 4, die aus einer thermoplastischen Kunstharzfolie hergestellt ist, und einen flüssigkeitssaugfähigen Kern 5, der aus einer Mischung von Faserpulpe und hochabsorbierendem Polymerpulver hergestellt ist und zwischen diesen beiden Lagen 3, 4 angeordnet ist. Die Deck- und die Außenlage 3, 4 sind in ihren nach außen über einen Umfangsrand des Kerns 5 hinausragenden Abschnitten wasserdicht miteinander verbunden, so daß in Längsrichtung einander gegenüberliegende Endklappen 6 gebildet werden, die keine Dehnbarkeit aufweisen, und in Querrichtung einander gegenüberliegende Seitenklappen 8.

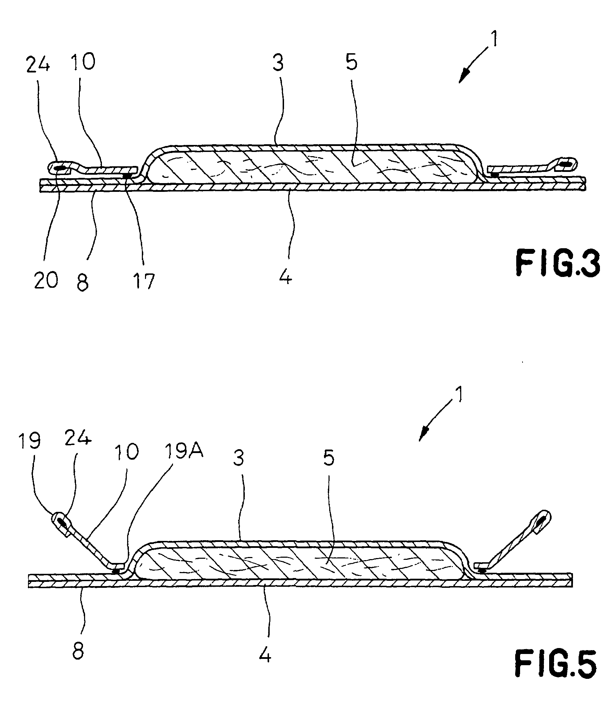

In Längsrichtung einandergegenüberliegende (in der Ansicht in Fig. 3 obere und untere) Endabschnitte 14, 15 der Binde 1 werden entlang gedachten Linien 12, 13, die die Binde 1 in Längsrichtung in drei im wesentlichen gleiche Teile teilen, nach innen gefaltet, so daß die Endabschnitte 14, 15 mit innenliegender Decklage 3 übereinander angeordnet werden können, und die auf diese Weise in drei Schichten gefaltete Binde 1 wird einzeln in der Umhüllung 2 verpackt, die durch eine unterbrochene Linie in Fig. 1 gezeigt ist. Eine Klappe 27 der Umhüllung 2 kann in der durch einen Pfeil A angegebenen Richtung geöffnet werden, um die Binde 1 aus dieser zu entnehmen.

Die Sperrklappe 10 umfaßt eine längliche Lage von flüssigkeitsundurchlässigem oder wasserabweisendem Vliesstoff, der aus thermoplastischen Synthetikfasern hergestellt ist, und ein elastisches Element 20, das unter Spannung mit der Innenseite eines Seitenrandes 19, der durch Falten der Lage gebildet ist, verklebt ist. Die Sperrklappe 10 ist somit durch den freien Seitenrand 19, einen dem freien Seitenrand 19 gegenüberliegenden Seitenrand 19A, der mit der Klappe 8 mittels linear aufgetragenem Heißschmelzkleber 17 verbunden ist, in Längsrichtung einander gegenüberliegende, nicht dehnbare Endabschnitte 22, 23, die an der Klappe 8 befestigt sind, und einen dehnbaren Mittelabschnitt 24, der sich zwischen diesen in Längsrichtung einander gegenüberliegenden Endabschnitten 22, 23 erstreckt, gebildet. Die Klappe 8 und die dieser Klappe 8 zugehörige Sperrklappe 10 werden durch Heißprägen derselben an den in Längsrichtung gegenüberliegenden Endabschnitten 22, 23 der Sperrklappe 10 miteinander verschmolzen, um ihre Starrheit zu verbessem, so daß die Endabschnitte 22, 23 sich auch dann nicht zusammenziehen, wenn diese Endabschnitte das elastische Element 20 enthalten.

Wie aus Fig. 2 ersichtlich ist, erstrecken sich die Linien 12, 13, entlang welchen die Binde 1 in drei Lagen gefaltet wird, in Querrichtung über die Sperrklappe 10 in ihrem Mittelabschnitt24 bevorzugt in einem Abstand von 10 Millimetern oder mehr von den inneren Enden 22A, 23A der einander in Längsrichtung gegenüberliegenden Endabschnitte 22 bzw. 23.

Fig. 3 zeigt bei horizontal entfalteter Binde 1 den Mittelabschnitt 24 der Klappe 10 in flachem Zustand.

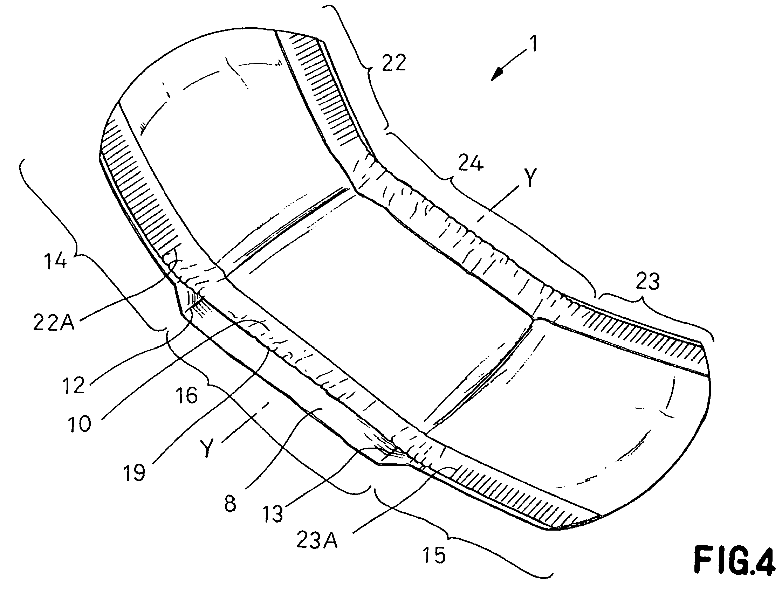

Fig. 4 zeigt die Binde 1, die aus der Umhüllung 2 entnommen wurde und auf einer horizontalen Oberfläche angeordnet wurde. Auch nachdem die Binde 1 entfaltet wurde, verbleibt entlang den Linien 12, 13 eine Restspannung der Faltung, und wenn ein zwischen den Faltlinien 12, 13 verlaufender Abschnitt horizontal angeordnet wird, erheben sich die in Längsrichtung einander gegenüberliegenden Endabschnitte 14, 15 leicht an den Faltlinien 12, 13. In diesem Zustand der Binde 1 wird der Abstand in einer geraden Linie zwischen den jeweiligen inneren Enden 22A, 23A der in Längsrichtung einander gegenüberliegenden Endabschnitte 22,23 der Klappe 10 kürzer als bei der horizontal entfalteten (Fig. 2) Binde 1, und der Mittelabschnitt 24 der Klappe 10 zieht sich entsprechend zusammen. Folglich erheben sich der Seitenrand 19 und der diesem Seitenrand 19 benachbarte Teil auf der hautberührenden Oberfläche der Binde 1.

Wie Fig. 5 zeigt, erhebt sich die Klappe 10 in ihrem Mittelabschnitt wie auch in dem diesem benachbarten Abschnitt, um eine Sperre zu bilden, die dazu dient, zu verhindern, daß Menstruationsflüssigkeit seitlich austritt. Während die Anordnung so dargestellt ist, daß der Seitenrand 19 außen liegt und der dem Seitenrand gegenüberliegende Seitenrand 19A innen liegt, ist es auch möglich, eine Anordnung in der Weise zu treffen, daß der Seitenrand 19 innen liegt. Eine Längsabmessung des Mittelabschnitts 24 ist bevorzugt 37 bis 70% und bevorzugter 40 bis 65% bezüglich der Gesamtlänge der Binde 1. Beträgt sie weniger als 37%, ist die Gesamtlänge des Mittelabschnitts 24 im wesentlichen gleich oder geringer als der Abstand zwischen den Faltlinien 12, 13, und es ist sehr schwierig oder nahezu unmöglich, den erwarteten Effekt der Faltung der Binde 1 zu erzielen. Wenn sie andererseits 70% oder mehr beträgt, ist es erforderlich, das elastische Element 20 mit der Klappe 10 mit einer relativ hohen Spannung zu verbinden, daß heißt einem relativ hohen Dehnungsverhältnis, um eine ausreichende Kontraktion des Mittelabschnitts 24 zu erreichen, oder dieser Mittelabschnitt 24 wird lose und erhebt sich oftmals nicht. Ein erhöhtes Dehnungsverhältnis beeinträchtigt jedoch in nachteiliger Weise die Weichheit und reizt in unangenehmer Art und Weise den empfindlichen Körperteil der Trägerin.

Durch Verwendung der Restspannung der Faltung, die in der Binde 1 entlang den Linien 12, 13 verbleibt, zum Zusammenziehen des Mittelabschnitts 24 der Sperrklappe 10 kann eine wirksame Sperre zuverlässig gebildet werden. Mit anderen Worten kann die Kontraktionskraft der Klappe 10 in ausreichender Weise eingespartwerden, um die Berührung der Klappe 10 mit dem empfindlichen Körperteil einer Trägerin abzumildern und dadurch den Tragekomfort zu verbessern.

Für die einzeln verpackte Damenbinde 1 gemäßder Erfindung können die Deck- und die Außenlage 3, 4 sowie der Kern 5 aus Materialien hergestellt sein, die gewöhnlich in diesem Industriezweig zur Herstellung dieser Elemente verwendet werden. Das Verbinden oder Befestigen der jeweiligen Elemente kann unter Verwendung von Heißschmelzkleber oder Heißsiegeltechniken erzielt werden und eine derartige Verbindungstechnik kann anstelle des vorstehend genannten Heißprägens verwendet werden. Auch ist es möglich, ohne den Schutzbereich der Erfindung zu verlassen, die Klappe 10 durch ein Gummituch oder einen dehnbaren Vliesstoff zu bilden und dadurch die Verwendung des elastischen Elements 20 zu eliminieren oder die Strecke zu begrenzen, über welche das elastische Element 20 mit dem Mittelabschnitt 24 verbunden werden sollte.

Obgleich nicht dargestellt, kann wenigstens der Kern 5 auf seiner oberen Fläche mit eingepreßten Nuten versehen sein, die jeweils einen konkaven (vorzugsweise V-förmigen) Querschnitt haben und entlang den jeweiligen Faltlinien 12, 13 verlaufen, nicht nur um die Kontraktion der Sperrklappe 10 zu unterstützen, sondern auch um eine diffusionsbedingte Permeation der Menstruationsflüssigkeit zu den in Längsrichtung einander gegenüberliegenden Endabschnitten 14, 15 zu unterdrücken. Ferner kann der Mittelabschnitt 16 ebenfalls wenigstens entlang seiner Mittellinie mit einer ähnlichen eingepreßten Nut versehen sein, die mit den Quemuten, die in der Oberfläche des Kerns 5 ausgebildet sind, verbunden ist, um die vorstehend genannte Funktion zu verbessern.

Gemäß der Erfindung wird die Damenbinde 1 entlang den quer über den dehnbaren Abschnitt jeder Sperrklappe verlaufenden Linien in drei Schichten gefaltet und einzeln in der Umhüllung verpackt, so daß die Restspannung der Faltung zur Erleichterung der Kontraktion des dehnbaren Abschnitts wirkt und dadurch zur zuverlässigen Formung der Sperre, die das seitliche Austreten von Menstruationsflüssigkeit verhindert.

## Patentansprüche

1. Einzeln verpackte Damenbinde (1), umfassend eine flüssigkeitsdurchlässige Decklage (3), eine flüssigkeitsundurchlässige Außenlage (4), einen zwischen diesen beiden Lagen angeordneten flüssigkeitssaugfähigen Kern (5) und ein Paar in Längsrichtung dehnbare Sperrklappen (10), die auf beiden Seiten der Binde (1) vorgesehen sind, wobei die Binde (1) durch Zurückfalten von in Längsrichtung einander gegenüberliegenden Endabschnitten (14, 15) derselben entlang quer zu der Binde (1) verlaufenden Faltlinien (12, 13) in drei Schichten gefaltet ist und anschließend in einer Umhüllung (2) verpackt ist, wobei
jede der Sperrklappen (10) einen in Längsrichtung dehnbaren Abschnitt (24) und nicht dehnbare Abschnitte (22, 23) umfaßt, die sich kontinuierlich von in Längsrichtung einander gegenüberliegenden Enden des dehnbaren Abschnitts (24) erstrecken
**dadurch gekennzeichnet,**
**daß** der dehnbare Abschnitt eine Länge von 37 bis 70% bezüglich der Gesamtlänge der Damenbinde (1) hat und die Faltlinien (12, 13) sich quer über den dehnbaren Abschnitt (24) erstrecken, wobei die Binde (1) nicht dehnbare Seitenklappen (8) hat, die durch Abschnitte der Decklage (3) und/oder der Außenlage (4) gebildet werden, die sich nach außen über in Querrichtung einander gegenüberliegende Seitenränder des Kerns (5) hinaus erstrecken und bei welcher in Längsrichtung einander gegenübeffiegende Endabschnitte (22, 23) der Sperrklappen (10) einstückig mit jeder der Seitenklappen (8) verbunden sind, um so nicht dehnbare Abschnitte (22, 23) zu bilden.

2. Damenbinde (1) nach Anspruch 1, bei welcher die Faltlinien (12, 13) 10 mm oder mehr von den Grenzen zwischen dem dehnbaren Abschnitt (24) und den nicht dehnbaren Abschnitten (22, 23) jeweils entfernt sind.

3. Damenbinde (1) nach Anspruch 1, bei welcher wenigstens der Kern (5) auf seiner oberen Fläche mit eingepreßten Nuten versehen ist, die sich entlang jeder der Faltlinien (12, 13) erstrecken.

## Claims

1. Individually packed sanitary napkin (1), comprising a liquid-permeable top sheet (3), a liquid-impermeable backsheet (4), a liquid absorbent core sandwiched between these two sheets and a pair of sealing flaps (10) extendible in longitudinal direction provided on both sides of the napkin (1), the napkin (1) being folded back into three layers by folding in longitudinal direction opposite end sections (14, 15) thereof at folding lines (12, 13) extending in transverse direction of the napkin (1), and subsequently being packed in a sheath (2),
with each of the sealing flaps comprising a section (24) extendible in longitudinal direction and sections (22, 23) not extendible, which extend continuously from opposite ends in the longitudinal direction of the extendible section (24),
**characterized in that**
the extendible section has a length of 37 to 70 % with respect to the total length of the sanitary napkin (1) and the folding lines (12, 13) extend across the extendible section (24), the sanitary napkin (1) having not extendible side flaps (8) formed by sections of the topsheet (3) and/or the backsheet (4), which extend outward beyond side edges of the core (5) opposite to each other in transverse direction, and wherein end sections (22, 23) of the sealing flaps (10) opposite to each other in longitudinal direction are continuously connected to each of the side flaps (8) so as to form non-extendible sections (22, 23).

2. Sanitary napkin (1) according to claim 1, wherein the folding lines (12, 13) are each in a distance of 10 mm or more from the borderline between the extendible section (24) and the non-extendible sections (22, 23).

3. Sanitary napkin according to claim 1, wherein at least the core (5) on its upper surface is provided with engraved grooves extending along each of the folding lines (12, 13).

## Revendications

1. En emballage unitaire, une serviette hygiénique pour dame (1), se composant d'une couche de couverture perméable au liquide (3), une couche extérieure imperméable au liquide (4), un noyau absorbant (5) placé entre ces deux couches et deux abattants de verrouillage (10) extensibles longitudinalement et prévus de part et d'autre de la serviette hygiénique (1) laquelle, par repli des extrémités (14, 15) en vis-à-vis dans le sens de la longueur, est pliée en trois couches suivant les lignes de pli (12, 13) transversales à la serviette (1) puis emballée dans une enveloppe (2),
chacun des abattants de verrouillage (10) comportant une section extensible dans le sens de la longueur (24) ainsi que des sections non extensibles (22, 23), qui s'étendent de manière continue dans le sens de la longueur par rapport aux extrémités opposées de la section extensible (24)
**caractérisée en ce**
**que** la longueur de la section extensible varie de 37 à 70% par rapport à la longueur totale de la serviette hygiénique (1) et que les lignes de pli (12, 13) transversales sont situées dans la section extensible (24), la serviette hygiénique (1) étant munie d'abattants latéraux (8) formés par des sections de la couche de couverture (3) et/ou de la couche extérieure (4), qui se prolongent vers l'extérieur transversalement par rapport aux bords latéraux du noyau (5), les sections non extensibles (22, 23) en vis-à-vis dans le sens de la longueur des abattants de blocage (10) étant reliés en une pièce avec chacun des abattants latéraux, de manière à former les sections non extensibles (22, 23).

2. Une serviette hygiénique pour dame (1) selon la revendication 1, dont les lignes de pli (12, 13) sont distantes de 10 mm ou davantage par rapport aux limites entre la section extensible (24) et les sections non extensibles (22, 23).

3. Une serviette hygiénique pour dame (1) selon la revendication 1, dont au moins le noyau (5) est muni sur la surface du dessus de rainures estampées partant le long de chacune des lignes de pli (12, 13).
